# EUROPÄISCHE PATENTSCHRIFT

(11) **EP 3 638 360 B1**
(45) Veröffentlichungstag und Bekanntmachung des Hinweises auf die Patenterteilung: **11.05.2022**
(21) Anmeldenummer: 18730760.8
(22) Anmeldetag: 11.06.2018
(51) Int. Cl.: A61N 1/32, A61N 2/02, A61N 5/06

(54) **VORRICHTUNG ZUR ERZEUGUNG VON ELEKTRISCHEN, MAGNETISCHEN UND/ODER ELEKTROMAGNETISCHEN SIGNALEN ZUR BEHANDLUNG DES MENSCHLICHEN KÖRPERS**
DEVICE FOR GENERATING ELECTRICAL, MAGNETIC AND/OR ELECTROMAGNETIC SIGNALS FOR TREATING THE HUMAN BODY
DISPOSITIF POUR PRODUIRE DES SIGNAUX ÉLECTRIQUES, MAGNÉTIQUES ET/OU ÉLECTROMAGNÉTIQUES DESTINÉS À TRAITER LE CORPS HUMAIN

(30) Priorität: 16.06.2017 DE 102017113259
(43) Veröffentlichungstag der Anmeldung: 22.04.2020
(73) Patentinhaber: Healy International AG, 16818 Kränzlin (DE)
(72) Erfinder: SCHMIEKE, Marcus, 16818 Kränzlin (DE); HILBURG, Andreas, 46045 Oberhausen (DE); KRZIZAN, Matthias, 06268 Nemsdorf-Göhrendorf (DE)
(74) Vertreter: Freischem & Partner Patentanwälte mbB
(86) Internationale Anmeldenummer: PCT/EP2018/065337
(87) Internationale Veröffentlichungsnummer: WO 2018/228987

(56) Entgegenhaltungen:
- DE-A1- 10 002 251
- DE-A1-102015 002 565
- DE-U1- 29 709 094
- US-A1- 2006 064 139

## Beschreibung

Die Erfindung betrifft eine Vorrichtung zur Erzeugung von elektrischen, magnetischen und/oder elektromagnetischen Signalen zur Behandlung des menschlichen Körpers.

Aus der Praxis sind Vorrichtungen zur Erzeugung von elektrischen, magnetischen und/oder elektromagnetischen Signalen zur Behandlung des menschlichen Körpers bekannt, insbesondere in Form von Elektrostimulationsgeräten. Solche Vorrichtungen können in den meisten Fällen mit verschiedenen Frequenzen betrieben werden. Der Anwendungserfolg der Vorrichtungen hängt in vielen Fällen davon ab, dass eine - für die jeweilige Einzelfallkonstellation - geeignete Behandlungsfrequenz ermittelt und angewendet wird. Dazu wird häufig ein Spezialist aufgesucht, der über entsprechende Erfahrung verfügt und auf der Grundlage seiner Erfahrung und unter Berücksichtigung der Umstände des jeweiligen Einzelfalls eine geeignete Behandlungsfrequenz ermittelt und anwendet oder zur Anwendung empfiehlt. Insbesondere wird auf die aus der Praxis bekannten Vorrichtungen verwiesen, die unter der Marke Timewaver in verschiedenen Ausführungen vertrieben werden. Nur beispielhaft wird insoweit Bezug genommen auf die Produkte "Timewaver Frequency" und "Timewaver Home". Die Erfindung bezieht sich insbesondere auf eine Weiterentwicklung solcher Produkte. Auf sämtliche technischen Merkmale dieser Produkte wird daher hiermit ausdrücklich verwiesen, insbesondere auf diejenigen Merkmale, welche bei allen genannten Produkten realisiert sind.

Aus DE 10 2015 002 565 A1 ist ein System zur Steuerung von Stimulations-Impulsen während einer Stimulation an einem Nutzer bekannt, die wenigstens einen Sensor, wenigstens eine Datenverarbeitungseinheit und wenigstens eine Impulseinheit aufweist. Als geeignete Sensoren werden sehr viele unterschiedliche Typen genannt, unter anderem Nahinfrarotspektroskopie(NIRS)-Sensoren. Bei diesem System soll die Datenverarbeitungseinheit konfiguriert sein, einen von einem Sensor gemessenen Wert mit einem Schwellenwert zu vergleichen und ein Steuersignal an die Impulseinheit zu generieren, wenn der Messwert und der Schwellenwert in einem vordefinierbaren Verhältnis zueinander stehen.

Aus DE 297 09 094 U1 ist ein Gerät für die Bioresonanz-Therapie zum therapeutische Zuführen elektromagnetischer Therapie-Signale zu einem Körper mit einer elektrischen Schaltung zur Aufnahme körpereigener oder stoffeigener Eingangssignale mittels einer Empfangsantenne oder Empfangselektrode bekannt. Dabei soll auch eine Signalverarbeitungsstufe zum Verarbeiten der Signale einer Ausgangsschaltung zum Zuführen der Ausgangssignale an eine Sendeantenne oder Sendeelektrode vorgesehen sein, wobei die Signalverarbeitungsstufe eine Verzögerungsschaltung aufweist, mittels welcher Ausgangssignale gegenüber den Eingangssignalen zeitlich verzögerbar sind.

Aus DE 100 02 251 A1 ist ein interaktives Wellnessgerät zur ganzheitlichen Sinnesansprache des Menschen bekannt, dessen Funktionen zumindest mittelbar durch gemessene Körperzustände des benutzenden Menschen gesteuert und moduliert werden.

Aus US 2006/0064139 A1 ist ein elektrisches Stimulationsgerät bekannt, das zur Einleitung von Frequenzen zwischen 1 Hz und 50 Hz über den Nervus Vagus in die Ohren eines Benutzers ausgebildet ist.

Der Erfindung liegt die Aufgabe zugrunde, eine Vorrichtung zur Erzeugung von elektrischen, magnetischen und/oder elektromagnetischen Signalen zur Behandlung des menschlichen Körpers zur Verfügung zu stellen, die eine neue und einfach anzuwendende Möglichkeit bietet, eine geeignete Behandlungsfrequenz auszuwählen.

Die Lösung der Aufgabe erfolgt erfindungsgemäß mit den Merkmalen des unabhängigen Anspruchs 1. Weitere praktische Ausführungsformen sind in Verbindung mit den abhängigen Ansprüchen beschrieben.

Bei einer erfindungsgemäßen Vorrichtung zur Erzeugung von elektrischen, magnetischen und/oder elektromagnetischen Signalen, die mit unterschiedlichen Behandlungsfrequenzen zur Behandlung des menschlichen Körpers einsetzbar sind, ist als Mittel zur Bereitstellung eines für die Auswahl einer Behandlungsfrequenz verwendbaren Rauschsignals ein elektronisches Rauschelement vorgesehen, dessen Eigenschaften eine zumindest teilweise Abhängigkeit von mindestens einer biophysikalischen Abstrahlung des menschlichen Körpers aufweisen, wobei als elektronisches Rauschelement im Sinne der Erfindung jedes elektronische Bauelement angesehen wird, das neben einem Ausgangssignal ein dem Ausgangssignal überlagertes Rauschsignal ausgibt und wobei das verwendbare Rauschsignal individuell, insbesondere aufgrund einer biophysikalischen Abstrahlung eines Körpers eines zu behandelnden Menschen, wie z.B. der Infrarotstrahlung, beeinflusst werden kann, so dass durch Wechselwirkung zwischen einem menschlichen Körper einer zu behandelnden Person und eines daraus resultierenden, durch die entsprechende Abstrahlung des menschlichen Körpers (z.B. Infrarotstrahlung) beeinflussten Rauschens eine geeignete Auswahl einer Behandlungsfrequenz erfolgen kann, wobei zur Auswertung des Rauschsignals Mittel zur Signalaufbereitung und zur Erzeugung eines digitalen Datenstroms des Rauschsignals vorgesehen sind und wobei zur Auswertung des Rauschsignals Mittel zur statistischen Auswertung des digitalen Datenstroms und Mittel zur Bestimmung einer Behandlungsfrequenz abhängig von der statistischen Auswertung vorgesehen sind.

Mit dem Begriff biophysikalische Abstrahlung sind alle insbesondere alle Feld-, Strahlungs- und Energieformen gemeint, die einen menschlichen Körper permanent umgeben oder temporär umgeben können, beispielsweise magnetische Felder, elektrische Felder, elektromagnetische Felder, Infrarotstrahlung, Wärmestrahlung, akustische Energie und/oder mechanische Energie. Jegliche biophysikalische Abstrahlung, welche derartige Felder, Strahlungen und/oder Energien umfasst, kann - einzeln oder in Kombination mehrerer Felder, Strahlungen und/oder Energien - die Eigenschaften eines für die Erfindung geeigneten Rauschelements beeinflussen.

Schon an dieser Stelle wird insbesondere auf Abstrahlung in Form von Infrarotstrahlung verwiesen. Infrarotstrahlung umfasst den Spektralbereich mit Wellenlängen zwischen 1 mm und 780 nm, was einem Frequenzbereich von 300 GHz bis 400 THz entspricht.

Für die Erfindung sind insbesondere solche elektronischen Rauschelemente geeignet, deren Eigenschaften eine Abhängigkeit in dem Spektralbereich aufweisen, in welchem der menschliche Körper Infrarotstrahlung oder andere biophysikalische Abstrahlung aussendet, so dass eine Vorrichtung, die in die Nähe des menschlichen Körpers gebracht wird, von dieser Abstrahlung des menschlichen Körpers beeinflusst wird.

Infrarotstrahlung des menschlichen Körpers findet insbesondere in den Bereichen des Nahinfrarot (NIR) mit Wellenlängen von 0,78 µm bis 1,4 µm (IR-A) und 1,4 µm bis 3,0 µm (IR-B) sowie im Bereich des mittleren Infrarot (MIR) mit Wellenlängen von 3,0 µm bis 50,0 µm statt. Besonders bevorzugt sind insoweit solche elektronischen Rauschelemente, die vor allem oder ausschließlich in den vorstehend genannten speziellen Bereichen eine Abhängigkeit von der entsprechenden Infrarotstrahlung aufweisen. Explizit verwiesen wird noch auf den Bereich mit Wellenlängen von 10,0 µm ± 9,0 µm, d.h. auf den Bereich zwischen 1,0 µm und 19,0 µm, weiter bevorzugt auf den Bereich zwischen 5,0 µm und 15 µm.

Als elektronisches Rauschelement im Sinne der Erfindung wird jedes elektronische Bauelement angesehen, das neben einem Ausgangssignal ein dem Ausgangssignal überlagertes Rauschsignal ausgibt. Diesbezüglich wird im Rahmen dieser Offenbarung unterschieden zwischen aktiven Rauschelementen und passiven Rauschelementen.

Als passives Rauschelement im Sinne der Erfindung werden solche elektronischen Bauelemente verstanden, die ohne eine externe Energieversorgung, wie beispielweise einer Spannungsquelle in Form eines Stromkabels oder einer Batterie, ein messbares Rauschsignal erzeugt. Diesbezüglich wird insbesondere auf den p-n-Übergang von Infrarot-Dioden, Transistoren oder anderen Bauelementen verwiesen, die unabhängig von den vorstehend genannten Energieversorgungen ein entsprechendes Rauschen erzeugen können. Der Vollständigkeit halber wird darauf verwiesen, dass auch passive Rauschelemente optional mit Energie versorgt und insbesondere mit einem Hilfsstrom beaufschlagt werden können. Für erfindungsgemäße Vorrichtungen sind solche elektronischen Rauschelemente besonders geeignet, die durch Beaufschlagung mit einem Hilfsstrom von weniger als 100 µA eine erkennbare Vergrößerung des Rauschbereiches erzeugen, insbesondere solche Rauschelemente, die mit weniger als 50 µA oder weniger als 30 µA Hilfsstrom eine entsprechende Vergrößerung von Frequenz und/oder Amplitude des Rauschens zu vergrößern.

Als aktive Rauschelemente im Sinne der Erfindung werden solche Elemente angesehen, die zur Erzeugung eines Rauschsignals zwangsläufig mit externer Energie in Form einer Versorgungsspannung bzw. eines entsprechenden Stromes beaufschlagt werden müssen, d.h. einen externen Energiespeicher benötigen. Diesbezüglich wird beispielhaft auf Kohleschichtwiderstände verwiesen.

Grundsätzlich ist die Auswertung des Rauschsignals auf beliebige Art und Weise möglich. In der Praxis hat es sich als besonders geeignet herausgestellt, zur Auswertung des Rauschsignals Mittel zur Signalaufbereitung und zur Erzeugung eines digitalen Datenstroms des Rauschsignals einzusetzen. Diesbezüglich wird insbesondere auf Mittel zur Erzeugung eines sehr einfachen digitalen Datenstroms in Form einer 1-Bit-Datenfolge, die ausschließlich aus Ergebnissen in Form einer 0 oder einer 1 besteht.

In diesem Zusammenhang wird insbesondere auf die Möglichkeit verwiesen, das Nutzspektrum des elektronischen Rauschelements, insbesondere durch Verwendung des Mittels zur Signalaufbereitung und zur Erzeugung eines digitalen Datenstroms, zu dämpfen und das Rauschspektrum zu verstärken.

So kann eine höhere Auflösung des Rauschens erzeugt werden.

In der beschriebenen Vorrichtung sind Mittel zur statistischen Auswertung des digitalen Datenstroms und Mittel zur Bestimmung einer Behandlungsfrequenz abhängig von der statistischen Auswertung vorgesehen. In diesem Zusammenhang ist zu berücksichtigen, dass die statistische Auswertung aufgrund der zumindest teilweisen Abhängigkeit des Rauschelements von Infrarotstrahlung abhängig von der Anordnung einer erfindungsgemäßen Vorrichtung relativ zu einem Infrarotstrahlung aussendenden menschlichen Körpers ist. Mit anderen Worten ausgedrückt, ist die Auswahl der Behandlungsfrequenz direkt abhängig von der Positionierung einer erfindungsgemäßen Vorrichtung in der Nähe eines menschlichen Körpers, insbesondere wenn die Vorrichtung so nah an einem menschlichen Körper positioniert ist, dass die von dem menschlichen Körper ausgehende Infrarotstrahlung das elektronische Rauschelement unmittelbar erreicht und somit dessen Eigenschaften beeinflusst.

Eine erfindungsgemäße Vorrichtung hat den Vorteil, dass ein für die Auswahl einer Behandlungsfrequenz verwendbares Rauschsignal individuell, insbesondere aufgrund einer biophysikalischen Abstrahlung eines Körpers eines zu behandelnden Menschen, wie z.B. der Infrarotstrahlung, beeinflusst werden kann, so dass durch Wechselwirkung zwischen einem menschlichen Körper einer zu behandelnden Person und eines daraus resultierenden, durch die entsprechende Abstrahlung des menschlichen Körpers (z.B. Infrarotstrahlung) beeinflussten Rauschens eine geeignete Auswahl einer Behandlungsfrequenz erfolgen kann.

Insbesondere betrifft die Erfindung kompakte, in unmittelbarer Nähe des Körpers positionierbare und betreibbare Vorrichtungen (sogenannte Wearables), die am Körper mitführbar und mittels portabler Energiespeicher betreibbar sind.

Die folgenden Merkmale einer erfindungsgemäßen Vorrichtung sind - sowohl einzeln als auch in beliebigen Kombinationen miteinander - bei vorteilhaften Ausführungsformen realisiert:
a) Eine erfindungsgemäße Vorrichtung weist vorzugsweise einen Anschluss für einen portablen Energiespeicher in Form einer Batterie auf. Der Energiespeicher kann fest installiert sein und/oder zum Austauschen oder Aufladen entnehmbar sein.
b) Die Vorrichtung weist vorzugsweise eine Größe von weniger als 20 cm x 10 cm x 5 cm auf, bevorzugt eine Größe von weniger als 10 cm x 10 cm x 3 cm und besonders bevorzugt, eine Größe von weniger als 8 cm x 8 cm x 2 cm.
c) Die Vorrichtung weist vorzugsweise Anschlüsse für mindestens zwei Pole (+/-) zum Anschließen von Elektroden auf, um elektrische Signale über solche Elektroden in einen menschlichen Körper einleiten zu können. Als geeignete Elektroden wird insbesondere auf Ohrclips, Klebeelektroden und Handelektroden verwiesen.
d) Die Vorrichtung weist vorzugsweise Anschlüsse für mindestens zwei Pole (+/-) zum Anschließen von Einrichtungen zur Aussendung elektromagnetischer Signale auf. Diesbezüglich wird insbesondere auf die Möglichkeit des Anschlusses von Magnetfeldspulen verwiesen.
e) Die Vorrichtung weist vorzugsweise einen Anschluss zum Laden eines portablen Energiespeichers auf, insbesondere einen Anschluss in Form einer USB-Buchse oder in Form eines sonstigen Schraub- und/oder Steckverbinders.
f) Die Vorrichtung ist vorzugsweise dazu ausgelegt, mit Behandlungsstromstärken von 0 µA bis 4000 µA betrieben zu werden. Besonders bevorzugt ist die Vorrichtung dazu ausgelegt, mit Behandlungsstromstärken von 0 µA bis 1000 µA betrieben zu werden. Mit dem Begriff Behandlungsstromstärken werden in diesem Zusammenhang die Stromstärken bezeichnet, welche von einer erfindungsgemäßen Vorrichtung in einen zu behandelnden menschlichen Körper eingebracht werden können, d.h. die von der Vorrichtung ausgesendeten Ströme.
g) Die Vorrichtung ist vorzugsweise dazu ausgelegt, mit Behandlungsfrequenzen zwischen 0 Hz und 1 MHz betrieben zu werden.
h) Die Vorrichtung ist vorzugsweise dazu ausgelegt, Behandlungsspannungen zwischen 0 V und 24 V abgeben zu können, insbesondere Behandlungsspannungen zwischen 0 V und 12 V und besonders bevorzugt zwischen 0 V und 10 V.

Dabei ist als Mittel zur Bereitstellung eines für die Auswahl einer Behandlungsfrequenz verwendbaren Rauschsignals ein Sensor zur Erfassung eines elektrischen, magnetischen oder elektromagnetischen Feldes, ein Sensor zur Erfassung von Infrarotstrahlung oder ein Sensor zur Erfassung von Wärmestrahlung vorgesehen. Selbstverständlich können auch zwei, drei oder mehr solcher Sensoren vorgesehen sein. Mit allen vorstehend genannten Sensoren kann ein Rauschsignal für eine erfindungsgemäße Vorrichtung erzeugt werden. Dieses Rauschsignal kann als Grundlage für das im Folgenden noch beschriebene Verfahren verwendet werden. Vorteilhaft ist dabei, dass zumindest eine biophysikalische Abstrahlung des Menschen, insbesondere einer zu behandelnden Person, das Rauschsignal beeinflussen kann, wenn die Vorrichtung mit dem Sensorelement in die Nähe des menschlichen Körpers gebracht ist. So kann die behandelnde Person selbst zumindest teilweise als Steuer- oder Regelelement einer solchen Vorrichtung berücksichtigt werden, wodurch eine neuartige, individuelle Ermittlung von Behandlungsfrequenzen ermöglicht ist, die der biophysikalischen Abstrahlung der zu behandelnden Person Rechnung trägt.

Mit einem geeigneten optionalen Programm, auf das im Folgenden noch näher eingegangen wird, kann mit einer erfindungsgemäßen Vorrichtung Reaktionen des menschlichen Körpers auf eine unmittelbar vorausgegangene Behandlung mit einer erfindungsgemäßen Vorrichtung Rechnung getragen werden, so dass ein Regelungsverfahren zur Auswahl der Behandlungsfrequenz unter Berücksichtigung der biophysikalischen Abstrahlung durchgeführt werden kann.

Als Sensoren für magnetische Felder wird insbesondere auf Hall-Sensoren und auf Wiegand-Sensoren verwiesen.

Zur Erfassung und Berücksichtigung von elektrischen Feldern können als Sensoren insbesondere kapazitive Sensoren eingesetzt werden.

Als Sensoren für elektromagnetische Felder wird insbesondere auf magneto-induktive Sensoren verwiesen.

Zur Erfassung und Berücksichtigung von Infrarotstrahlung wird insbesondere auf Infrarot-Fotodioden und auf Pyrometer verwiesen.

Und zur Erfassung von Wärmestrahlung (MIR) wird auf Germanium-Fotodioden und Silizium Fotodioden verwiesen.

Der Vollständigkeit halber wird auch noch darauf hingewiesen, dass akustische Energie (Körperschall) insbesondere mit Hilfe eines Rauschelements in Form eines piezoelektrischen Sensors erfasst und berücksichtigt werden kann.

Das Gleiche gilt für mechanische Energie, die beispielsweise über halbleiterbasierende Dehnungsmessstreifen erfasst und berücksichtigt werden kann.

Wenn ein passives Rauschelement vorgesehen ist, hat dies den Vorteil, dass auch ohne eine externe Energieversorgung auf besonders effiziente und kostengünstige Art und Weise ein entsprechendes Rauschsignal zur Verfügung steht. Besonders geeignet sind passive Rauschelemente, die optional auch als aktive Rauschelemente betrieben werden können, insbesondere indem diese bedarfsweise mit einem wie vorstehend erläuterten Hilfsstrom beaufschlagt werden. Die Beaufschlagung mit einem entsprechenden Hilfsstrom kann insoweit von Vorteil sein, als die Realisierung einer entsprechenden Vorrichtung, insbesondere wenn das Rauschsignal für die vorgesehene Auswahl digitalisiert werden soll, zunächst aufbereitet werden muss, um eine entsprechende Auswahl der Behandlungsfrequenz auf geeignete Art und Weise zu treffen.

Insbesondere wird in diesem Zusammenhang auf die Möglichkeit verwiesen, als Mittel zur Bereitstellung eines für die Auswahl einer Behandlungsfrequenz verwendbaren Rauschsignals einen Sensor zur Erfassung von Infrarotstrahlung in Form einer Infrarot-Diode zu verwenden bzw. eine solche Infrarot-Diode in einer entsprechenden Vorrichtung vorzusehen, deren p-n-Übergang als passives Rauschelement dient. Solche Infrarot-Dioden stehen als elektronische Bauelemente nicht nur relativ kostengünstig zur Verfügung, weil sie in großen Mengen insbesondere auch für andere Anwendungszwecke hergestellt werden, sondern haben darüber hinaus den Vorteil, dass die Lebensdauer solcher Infrarot-Dioden sehr groß ist, insbesondere größer als die erwartete Lebens- bzw. Nutzungsdauer einer erfindungsgemäßen Vorrichtung. Es kann insoweit durch die Verwendung einer solchen Infrarot-Diode mit hoher Wahrscheinlichkeit davon ausgegangen werden, dass die erfindungsgemäße Vorrichtung in Bezug auf das Rauschsignal über einen langen Zeitraum sicher funktionieren wird.

In einer weiteren praktischen Ausführungsform einer erfindungsgemäßen Vorrichtung ist als Rauschsignal eine Rauschspannung vorgesehen. Damit ist gemeint, dass das sogenannte Rauschen einer als Ausgangssignal des Rauschelements dienenden Rauschspannung überwacht und als Rauschsignal der erfindungsgemäßen Vorrichtung für die Auswahl einer geeigneten Behandlungsfrequenz eingesetzt wird. Die Verwendung einer Rauschspannung als Rauschsignal stellt eine wirtschaftlich sinnvolle Alternative zur Verwendung einer Rauschstromstärke insoweit dar, als die Aufbereitung eines Signals in Form einer Rauschspannung technisch einfacher und somit kostengünstiger realisierbar ist. Dazu kann insbesondere ein hochfrequenter Operationsverstärker eingesetzt werden.

Die Erfindung kann auch für ein Verfahren unter Verwendung einer wie vorstehend beschriebenen, erfindungsgemäßen Vorrichtung eingesetzt werden. Dieses Verfahren ist jedoch kein Teil der Erfindung.

Dazu wird die Vorrichtung vor und/oder während der Verwendung einer entsprechenden Vorrichtung derart in Körpernähe eines zu behandelnden menschlichen Körpers gebracht, dass die biophysikalische Abstrahlung des menschlichen Körpers in Wechselwirkung mit dem elektronischen Rauschelement tritt, so dass die Auswahl der Behandlungsfrequenz in Abhängigkeit der biophysikalischen Abstrahlung des zu behandelnden menschlichen Körpers erfolgt. Hinsichtlich der Vorteile des Verfahrens wird auf die vorstehend bereits beschriebenen Vorteile in Verbindung mit der erfindungsgemäßen Vorrichtung verwiesen. Auch diesbezüglich wird noch einmal explizit auf die biophysikalische Abstrahlung in Form von Infrarotstrahlung des menschlichen Körpers verwiesen.

In einer praktischen Ausführungsform eines solchen Verfahrens beträgt der Abstand der Vorrichtung zu dem behandelnden Körper vor und/oder während der Anwendung maximal 50 cm. Es wird davon ausgegangen, dass die meisten biophysikalischen Abstrahlungen eines menschlichen Körpers, insbesondere Infrarotstrahlung, in einem solchen Abstand noch Einfluss auf ein geeignetes Rauschelement in einer erfindungsgemäßen Vorrichtung nehmen kann. Da die Intensität der biophysikalischen Abstrahlung, insbesondere der Infrarotstrahlung, jedoch mit abnehmendem Abstand umso größer ist, ist es bevorzugt, wenn der Abstand der Vorrichtung zu dem behandelnden Körper maximal 25 cm, weiter bevorzugt maximal 10 cm und besonders bevorzugt maximal 5 cm beträgt. Dementsprechend wird die Vorrichtung gemäß dem Verfahren vorzugsweise direkt am Körper einer zu behandelnden Person getragen, so dass der Abstand zwischen dem Rauschelement und dem zu behandelnden menschlichen Körper minimiert ist.

In einer weiteren praktischen Ausführungsform des Verfahrens wird das elektronische Rauschelement mit einem Hilfsstrom beaufschlagt. Diesbezüglich wird noch einmal auf die bevorzugte Verwendung eines passiven Rauschelements in Form einer Infrarot-Diode oder eines Transistors mit einem entsprechenden p-n-Übergang verwiesen. Die Beaufschlagung eines solchen elektronischen Rauschelements mit einem Hilfsstrom hat den Vorteil, dass eine wie vorstehend bereits beschriebene Aufbereitung und Digitalisierung des Signals zur Erzeugung einer kurzfristigen und einfachen Auswahlentscheidung hinsichtlich der Behandlungsfrequenz einfach und kostengünstig realisiert werden kann.

Ein Verfahren kann derart durchgeführt werden, dass vor oder zu Beginn der Behandlung eines menschlichen Körpers zunächst wie vorstehend beschrieben eine geeignete Behandlungsfrequenz ermittelt und dann von der erfindungsgemäßen Vorrichtung eingestellt wird. Die Behandlung kann in diesem Fall über eine vorgegebene Zeitdauer mit der ermittelten Behandlungsfrequenz durchgeführt werden.

In einer anderen Variante des Verfahrens kann nach Ablauf einer gewissen Zeitdauer auch eine neue Behandlungsfrequenz ermittelt und von der Vorrichtung eingestellt werden. Die erfindungsgemäße Vorrichtung kann dazu eine entsprechende Steuerung aufweisen, in welcher ein entsprechendes Bedienprogramm umfasst ist, das einen Anwender einer erfindungsgemäßen Vorrichtung in der Durchführung eines entsprechenden Verfahrens unterstützt.

Ein wie vorstehend beschriebenes Verfahren kann insoweit auch so durchgeführt werden, dass nach einer voreingestellten Zeitdauer von beispielsweise 10 Sekunden, 20 Sekunden, 30 Sekunden oder einem beliebigen anderen Zeitdauerwert, der fest vorgegeben ist, eine neue Behandlungsfrequenz ermittelt und von der Vorrichtung eingestellt wird, so dass jeweils für eine vorgegeben Zeitdauer die Behandlung des menschlichen Körpers mit der jeweils ermittelten Behandlungsfrequenz erfolgt. Dazu können in einer erfindungsgemäßen Vorrichtung ein entsprechendes Programm oder mehrere Programme festhinterlegt sein und/oder die Vorrichtung kann entsprechend kann derart programmierbar gestaltet sein, dass der Benutzer der Vorrichtung selbst individuelle Programme konfigurieren und speichern kann.

Insbesondere in Verbindung den vorstehend erwähnten Programmen kann eine erfindungsgemäße Vorrichtung dazu geeignet sein, eine Regelung der Behandlungsfrequenz insoweit zu ermöglichen, als die Infrarotstrahlung des menschlichen Körpers einen Regelparameter für die Behandlungsfrequenz darstellt. Dabei handelt es sich bei der Infrarotstrahlung des menschlichen Körpers insoweit um einen Regelparameter (in Abgrenzung zu einem Steuerparameter), als davon auszugehen ist, dass eine Wechselwirkung sowohl zwischen der Behandlungsfrequenz und dem menschlichen Körper (einschließlich dessen Infrarotstrahlung) und eine Wechselwirkung zwischen dem menschlichen Körper (einschließlich der Infrarotstrahlung) und der Behandlungsfrequenz besteht. So kann insbesondere die - unter Berücksichtigung der anfänglichen Infrarotstrahlung des zu behandelnden menschlichen Körpers ermittelte Behandlungsfrequenz dazu führen, dass die Infrarotstrahlung des menschlichen Körpers sich - aufgrund der Einwirkung der elektrischen, magnetischen und/oder elektromagnetischen Signale in der ausgewählten Behandlungsfrequenz - während der Zeitdauer der ersten Behandlungsphase verändert und diese Veränderung wiederum zu einer weiteren Veränderung der nächsten Behandlungsfrequenz führt. Dies kann - abhängig vom Einzelfall - dazu führen, dass die Behandlungsfrequenz nach jeder Zeitdauer verändert wird oder auch dazu, dass die Behandlungsfrequenz sich bei einem bestimmten Frequenzwert "einschwingt", sich einem bestimmten Ziel-Frequenzwert sukzessive annähert oder aber auch nach jeder Zeitdauer einen völlig anderen Wert annimmt.

Wichtig ist, dass die biophysikalische Abstrahlung des menschlichen Körpers der behandelnden Person, hier insbesondere die Infrarotstrahlung, die Auswahl der Behandlungsfrequenz regeln oder steuern kann.

Von einem Steuern der Behandlungsfrequenz kann man insbesondere in den Fällen sprechen, in welchen anfänglich - wiederum unter Berücksichtigung der biophysikalischen Abstrahlung des menschlichen Körpers der behandelnden Person - eine Behandlungsfrequenz ermittelt wird und die Vorrichtung dann für eine gewisse Behandlungsphase beibehalten wird, so dass es zu keiner Rückkopplung einer etwaige sich verändernden biophysikalischen Abstrahlung, insbesondere Infrarotstrahlung, des menschlichen Körpers während der Behandlung kommt.

Weitere praktische Ausführungsformen und Vorteile der Erfindung sind nachfolgend im Zusammenhang mit den Zeichnungen beschrieben. Es zeigen:
- Fig. 1: eine schematische Darstellung einer erfindungsgemäßen Vorrichtung und
- Fig. 2: eine schematische Darstellung der Funktionsweise der in Fig. 1 gezeigten erfindungsgemäßen Vorrichtung während der Anwendung.

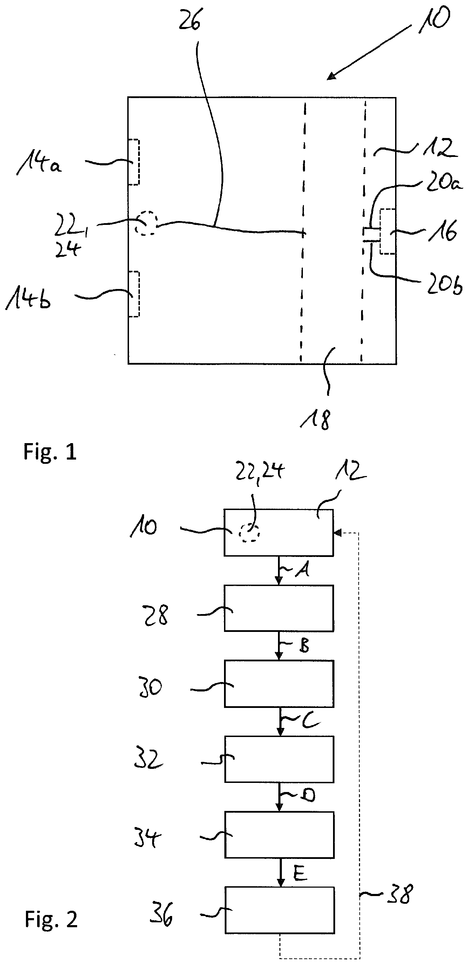

Fig. 1 zeigt eine schematische Darstellung einer erfindungsgemäßen Vorrichtung 10. Die Vorrichtung umfasst ein in seinen geometrischen Abmessungen relativ kleines Gehäuse 12, so dass es am Körper einer Person angenehm - und sofern gewünscht auch versteckt - getragen werden kann. Im gezeigten Ausführungsbeispiel betragen die Außenmaße des Gehäuses 12 5 cm (Länge) x 5 cm (Breite) x 1 cm (Tiefe).

Das Gewicht der dargestellten Vorrichtung ist geringer als 1 kg, insbesondere geringer als 800 g und bevorzugt geringer als 500 g. Es lässt sich damit aufgrund seiner Größe und seines Gewichts - auch über längere Zeiträume - von einer Person mitführen, ohne dass es dabei als große Gewichtsbelastung empfunden wird. Es handelt sich somit um ein sogenanntes "Wearable".

An dem Gehäuse 12 sind ein erster Anschluss 14a für einen Pluspol und ein zweiter Anschluss 14b für einen Minuspol vorgesehen. Über diese Anschlüsse 14a, 14b lassen sich wahlweise Elektroden (nicht gezeigt), Magnetspulen oder sonstige Zusatzgeräte anschließen, die zur kontaktbehafteten oder kontaktfreien Einleitung von elektrischen, magnetischen und/oder elektromagnetischen Signalen in einen menschlichen Körper einer zu behandelnden Person geeignet sein können.

An dem Gehäuse 12 ist ferner ein Strom- und Datenanschluss 16 für die Aufladung eines nur schematisch dargestellten Energiespeichers 18 vorgesehen, der im Inneren des Gehäuses 12 geschützt angeordnet und über elektrische Leitungen 20a, 20b mit dem Strom- und Datenanschluss 16 verbunden ist. Bei dem Energiespeicher 18 handelt es sich in diesem Ausführungsbeispiel um eine wiederaufladbare Batterie, bei dem Strom- und Datenanschluss 16 um einen USB-Anschluss.

In dem Gehäuse 12 ist ferner, hier nahe einer Außenseite des Gehäuses 12, als Mittel zur Bereitstellung eines für die Auswahl einer Behandlungsfrequenz verwendbaren Rauschsignals ein elektronisches Rauschelement 22 in Form einer Infrarot-Diode 24 angeordnet. Diese Infrarot-Diode 24 kann über eine Leitung 26 von dem Energiespeicher 18 mit einem Hilfsstrom beaufschlagt werden. In dem Gehäuse 12 ist ein - in Fig. 1 nicht dargestellter - Durchlassbereich in Form einer Aussparung und/oder eines für Infrarotstrahlung durchlässigen Gehäuseabschnitts, beispielsweise in Form eines Infrarot-Fensters.

Die Vorrichtung 10 umfasst weitere, nicht im Detail dargestellte Elemente, insbesondere einen Ein-/Ausschalter sowie optionale Bedientasten. Alternativ kann die Vorrichtung 10 auch einen Funksender aufweisen, der mit einer nicht dargestellten Steuerung verbunden ist und es ermöglicht, die Vorrichtung mit einem mobilen Endgerät drahtlos zu verbinden und zu steuern, insbesondere über ein Mobiltelefon (vorzugsweise ein Smartphone), ein Tablet-PC oder ein sonstiges mobiles Endgerät.

Fig. 2 zeigt die Funktionsweise einer erfindungsgemäßen Vorrichtung 10 in einer schematischen Darstellung während der Verwendung. Die Vorrichtung 10 ist in Fig. 2 stark abstrahiert nur mit dem in dem Gehäuse 12 angeordneten Rauschelement 22 dargestellt, das in dem Ausführungsbeispiel in Form einer Infrarot-Diode 24 vorliegt. Die übrigen Elemente, wie z.B. ein Energiespeicher etc. sind ebenfalls vorhanden, aber in Fig. 2 nicht dargestellt.

Wenn die Vorrichtung 10 in die Nähe einer nicht dargestellten Person, insbesondere einer mit der Vorrichtung 10 zu behandelnden Person, gebracht wird, wirkt die Infrarotstrahlung dieser Person unmittelbar auf das Rauschelement 22 ein und verändert die Eigenschaften des Rauschens. Voraussetzung dafür ist jedoch, dass der Abstand zwischen der Vorrichtung 10 und der Person ausreichend klein ist, insbesondere maximal 20 cm, bevorzugt maximal 10 cm und weiter bevorzugt maximal 5 cm.

Das - durch die Infrarotstrahlung beeinflusste - Rauschen des Rauschelements 22 wird nun in einem ersten Schritt A optional mittels eines Verstärkers 28 verstärkt. Dazu kann insbesondere ein bekannter Operationsverstärker dienen.

Mittels eines weiteren optionalen Schritts B wird das Rauschen dann in einem optionalen Schritt C einem AC/DC-Wandler 30 zugeführt und schließlich in einem weiteren optionalen Schritt D einem Filter 32 zugeführt.

Es folgt eine weitere Aufarbeitung des Rauschens in einem Schritt E mittels einer Digitalisiervorrichtung, insbesondere in Form eines Comparators 34, um ein digitalisiertes Rauschsignal in Form eines Bitstream zu erzeugen, bevor in einem letzten optionalen Schritt E mittels einer statistischen Auswerteeinheit 36 eine Auswahl einer Behandlungsfrequenz erfolgt.

Durch den gestrichelten Pfeil 38 ist angedeutet, dass die Behandlungsfrequenz, wenn sie auf den nicht dargestellten Körper der zu behandelnden Person angewendet wird, wiederum eine Rückkopplung auf eine - ggf. mit der Vorrichtung 10 - neu zu ermittelnde Behandlungsfrequenz haben kann.

Die Erfindung ist in den Ansprüchen definiert. Andere Beispiele, Ausführungsformen, Programmen und Verfahren sind keine Teile der Erfindung.

### Bezugszeichenliste

- 10: Vorrichtung
- 12: Gehäuse
- 14a, 14b: Anschluss
- 16: Strom- und Datenanschluss (USB)
- 18: Energiespeicher (Batterie)
- 20a, 20b: Leitung
- 22: Rauschelement
- 24: Infrarot-Diode
- 26: Leitung
- 28: Verstärker
- 30: AC/DC-Wandler
- 32: Filter
- 34: Comparator
- 36: Auswerteeinheit
- 38: Pfeil

## Patentansprüche

1. Vorrichtung zur Erzeugung von elektrischen, magnetischen und/oder elektromagnetischen Signalen, die mit unterschiedlichen Behandlungsfrequenzen zur Behandlung des menschlichen Körpers einsetzbar sind,
**dadurch gekennzeichnet,**
**dass** als Mittel zur Bereitstellung eines für die Auswahl einer Behandlungsfrequenz verwendbaren Rauschsignals ein elektronisches Rauschelement (22) vorgesehen ist, dessen Eigenschaften eine zumindest teilweise Abhängigkeit von mindestens einer biophysikalischen Abstrahlung des menschlichen Körpers aufweisen, wobei als elektronisches Rauschelement im Sinne der Erfindung jedes elektronische Bauelement angesehen wird, das neben einem Ausgangssignal ein dem Ausgangssignal überlagertes Rauschsignal ausgibt und wobei das verwendbare Rauschsignal individuell aufgrund einer biophysikalischen Abstrahlung eines Körpers eines zu behandelnden Menschen beeinflusst werden kann, so dass durch Wechselwirkung zwischen einem menschlichen Körper einer zu behandelnden Person und eines daraus resultierenden, durch die entsprechende Abstrahlung des menschlichen Körpers beeinflussten Rauschens eine geeignete Auswahl einer Behandlungsfrequenz erfolgen kann, wobei als als Mittel zur Bereitstellung eines für die Auswahl einer Behandlungsfrequenz verwendbaren Rauschsignals mindestens ein Sensor zur Erfassung eines elektrischen, magnetischen oder elektromagnetischen Feldes, ein Sensor zur Erfassung von Infrarotstrahlung oder ein Sensor zur Erfassung von Wärmestrahlung vorgesehen ist, wobei zur Auswertung des Rauschsignals Mittel zur Signalaufbereitung und zur Erzeugung eines digitalen Datenstroms des Rauschsignals vorgesehen sind und wobei zur Auswertung des Rauschsignals Mittel zur statistischen Auswertung des digitalen Datenstroms und Mittel zur Bestimmung einer Behandlungsfrequenz abhängig von der statistischen Auswertung vorgesehen sind.

2. Vorrichtung nach dem vorstehenden Anspruch, **dadurch gekennzeichnet, dass** als Mittel zur Bereitstellung eines für die Auswahl einer Behandlungsfrequenz verwendbaren Rauschsignals ein Sensor zur Erfassung von Infrarotstrahlung in Form einer Infrarot-Diode (24) vorgesehen ist, deren p-n-Übergang als passives Rauschelement (22) dient.

3. Vorrichtung nach einem der vorstehenden Ansprüche, **dadurch gekennzeichnet, dass** als Rauschsignal eine Rauschspannung vorgesehen ist.

## Claims

1. Device for generating electrical, magnetic and/or electromagnetic signals, which can be used with different treatment frequencies for treating the human body, **characterised in that**
as means of providing a noise signal that can be used for the selection of a treatment frequency, an electronic noise element (22) is provided, the properties of which have at least a partial dependence on at least one biophysical radiation of the human body, wherein any electronic component that, in addition to an output signal, outputs a noise signal that superimposes the output signal is considered as an electronic noise element within the meaning of the invention and wherein the usable noise signal can be influenced individually on the basis of a biophysical radiation of a body of a person to be treated such that a suitable selection of a treatment frequency can take place by an interaction between a human body of a person to be treated and a resulting noise influenced by the corresponding radiation of the human body, wherein at least one sensor for detecting an electrical, magnetic or electromagnetic field, a sensor for detecting infrared radiation or a sensor for detecting heat radiation is provided as a means for providing a noise signal that can be used for the selection of a treatment frequency, wherein means for signal processing and for generating a digital data stream of the noise signal are provided for evaluating the noise signal and wherein means for statistically evaluating the digital data stream and means for determining a treatment frequency depending on the statistical evaluation are provided for evaluating the noise signal.

2. The device according to the preceding claim, **characterised in that** as means for providing a noise signal that can be used for the selection of a treatment frequency, a sensor for detecting infrared radiation in the form of an infrared diode (24) is provided, the p-n-junction of which serving as a passive noise element (22).

3. The device according to any one of the preceding claims, **characterised in that** a noise voltage is provided as a noise signal.

## Revendications

1. Dispositif pour la production de signaux électriques, magnétiques et/ou électromagnétiques pouvant être utilisés à différentes fréquences de traitement pour traiter le corps humain,
**caractérisé en ce que**
un élément électronique générateur de bruit (22), dont les propriétés présentent une dépendance au moins partielle à au moins un rayonnement biophysique du corps humain, est prévu comme moyens de fourniture d'un signal de bruit pouvant être utilisé pour le choix d'une fréquence de traitement, où tout composant électronique qui, en plus d'un signal de sortie, émet un signal de bruit superposé au signal de sortie est considéré comme l'élément électronique générateur de bruit au sens de la présente invention et où le signal de bruit utilisable peut être influencé de manière individuelle sur la base d'un rayonnement biophysique d'un corps humain à traiter, de sorte qu'un choix approprié d'une fréquence de traitement peut avoir lieu grâce à l'interaction entre un corps humain d'une personne à traiter et un bruit résultant influencé par le rayonnement correspondant du corps humain, où au moins un capteur pour la détection d'un champ électrique, magnétique ou électromagnétique, un capteur pour la détection d'un rayonnement infrarouge ou un capteur pour la détection d'un rayonnement thermique fait office de moyens de fourniture d'un signal de bruit pouvant être utilisé pour le choix d'une fréquence de traitement, où des moyens de conditionnement de signal et de génération d'un flux de données numériques du signal de bruit sont prévus pour l'évaluation du signal de bruit, et où des moyens d'évaluation statistique du flux de données numériques et des moyens de détermination d'une fréquence de traitement en fonction de l'évaluation statistique sont prévus pour évaluer le signal de bruit.

2. Dispositif selon la revendication précédente, **caractérisé en ce qu'**un capteur pour la détection d'un rayonnement infrarouge sous la forme d'une diode infrarouge (24) dont la jonction p-n sert d'élément de bruit passif (22) est prévu comme moyens de fourniture d'un signal de bruit pouvant être utilisé pour le choix d'une fréquence de traitement.

3. Dispositif selon l'une quelconque des revendications précédentes, **caractérisé en ce qu'**il est prévu qu'une tension de bruit fasse office de signal de bruit.
